# EUROPÄISCHE PATENTSCHRIFT

(11) **EP 1 898 810 B1**
(45) Veröffentlichungstag und Bekanntmachung des Hinweises auf die Patenterteilung: **05.04.2017**
(21) Anmeldenummer: 06724725.4
(22) Anmeldetag: 04.05.2006
(51) Int. Cl.: A61B 90/00

(54) **APPLIKATOR**
APPLICATOR
APPLICATEUR

(30) Priorität: 01.06.2005 DE 102005025187
(43) Veröffentlichungstag der Anmeldung: 19.03.2008
(73) Patentinhaber: Luye Pharma AG, 83714 Miesbach (DE)
(72) Erfinder: BUSSMANN, Oliver, 83229 Aschau (DE)
(74) Vertreter: Forstmeyer, Dietmar
(86) Internationale Anmeldenummer: PCT/EP2006/004208
(87) Internationale Veröffentlichungsnummer: WO 2006/128548

(56) Entgegenhaltungen:
- EP-A- 1 312 315
- US-A- 4 474 572
- US-A1- 2003 040 766
- US-B1- 6 213 932

## Beschreibung

Die Erfindung betrifft einen Applikator zur Implantation eines Implantats, mit einem Zylinder und einem darin verschieblich gelagertem Stempel, welcher Stempel einen Druckstempel zum Hinausdrücken des Implantats aus einer Kanüle aufweist.

Bei den bekannten Applikatoren werden Implantate, die sich in einer Kanüle befinden, zur Ablage unter der Haut oder im Gewebe unter die Haut gestochen und dann aus der Kanüle mittels eines Druckstempels hinausgedrückt.

Dabei muss der Arzt zunächst die Kanüle des Applikators unter die Haut stechen und darauf unter Eindrücken des Stempels und gleichzeitigem Zurückziehen des Applikators eine problemlose Ablage für das Implantat möglich machen.

Diese genau zu koordinierende zweifache Bewegung - Drücken des Druckstempels durch Verschieben des Stempels unter entsprechendem Zurückziehen des ganzen Applikators samt Kanüle aus dem Gewebe - erfordert Geschick und Übung.

EP 1 312 315 A1 beschreibt eine Haartransplantationsvorrichtung mit einem Zylinder, einem Stempel und einem Getriebe, mit der ein Haartransplantat aus einem behaarten Bereich der Kopfhaut entnommen und in einen kahlen Bereich der Kopfhaut implantiert wird.

Der Erfindung liegt daher die Aufgabe zugrunde einen Applikator zur Verfügung zu stellen, der eine erleichterte Ablage eines Implantats ermöglicht.

Diese Aufgabe wird durch einen Applikator mit den Merkmalen des Anspruchs 1 gelöst.

Erfindungsgemäß ist nach der Erfindung vorgesehen, dass die Kanüle an einem zylinderförmigen Kanülenträger bzw. Zylinderabschnitt befestigt oder befestigbar ist, wobei der Zylinderabschnitt im Zylinder beweglich geführt ist, wobei ein Getriebe vorgesehen ist, das den Stempel mit dem Zylinderabschnitt koppelt, wobei eine Relativbewegung des Stempels gegenüber dem Zylinder in eine entgegengesetzte Bewegung des Zylinderabschnitts umgesetzt wird.

Hierdurch wird, durch das Getriebe gesteuert, durch das Hineindrücken des Stempels automatisch die Kanüle in einer Gegenbewegung zurückgezogen und so das Implantat in einem sich selbsttätig bildenden Hohlraum abgelegt.

Eine vorteilhafte und bevorzugte Ausgestaltung der Erfindung sieht vor, dass das Getriebe durch eine mit dem Stempel verbundene oder angeformte erste Zahnstange ausgebildet ist, die in Eingriff mit einem am Zylinder drehbar an einer Achse gelagertem Zahnrad steht, welches ebenfalls in Eingriff mit einer zweiten Zahnstange steht, die am Zylinderabschnitt befestigt oder angeformt ist.

Dem folgend ist eine Weiterbildung vorgeschlagen, wonach die Kupplung durch einen Luer-Lock ausgebildet ist.

Erfindungsgemäß ist eine den Zylinder wenigstens teilweise umschließende Hülse vorgesehen, wobei die Hülse um den Zylinder verschieblich gelagert ist und soweit über das kupplungsseitige Ende des Zylinders verschieblich ist, dass sie eine an der Kupplung des Applikators angekuppelte Kanüle wenigstens teilweise umschließt.

Die Hülse ist vorteilhafterweise in einer Zwangsführung an dem Zylinder geführt, wobei die Zwangsführung vorteilhafterweise durch eine Ausnehmung in der Hülse und eine darin in Eingriff stehende Ausformung an der Außenseite des Zylinders ausgebildet sein kann.

Weitere Vorteile, Besonderheiten und zweckmäßige Weiterbildungen der Erfindung ergeben sich aus den weiteren Unteransprüchen oder deren Unterkombinationen.

Nachfolgend wird die Erfindung anhand der Zeichnungen weiter erläutert. Im Einzelnen zeigt die schematische Darstellung in:
- Fig. 1: eine schematische Schnittdarstellung eines erfindungsgemäßen Applikators zur Implantation eines Implantats mit einem Zylinder und einem darin verschieblich gelagertem Stempel,
- Fig. 2: eine schematische Schnittdarstellung des Zylinders mit daran geführter Hülse, ohne den Stempel und den Zylinderabschnitt,
- Fig. 3: eine schematische Schnittdarstellung des Stempels mit daran angeformter Zahnstange und angeformtem Druckstempel, und
- Fig. 4: eine schematische Schnittdarstellung des Zylinderabschnitts mit daran angeformter Zahnstange und einem Luer-Lock als Kupplung für eine Kanüle.

Die in den Figuren gleichen Bezugsziffern bezeichnen gleiche oder gleich wirkende Elemente.

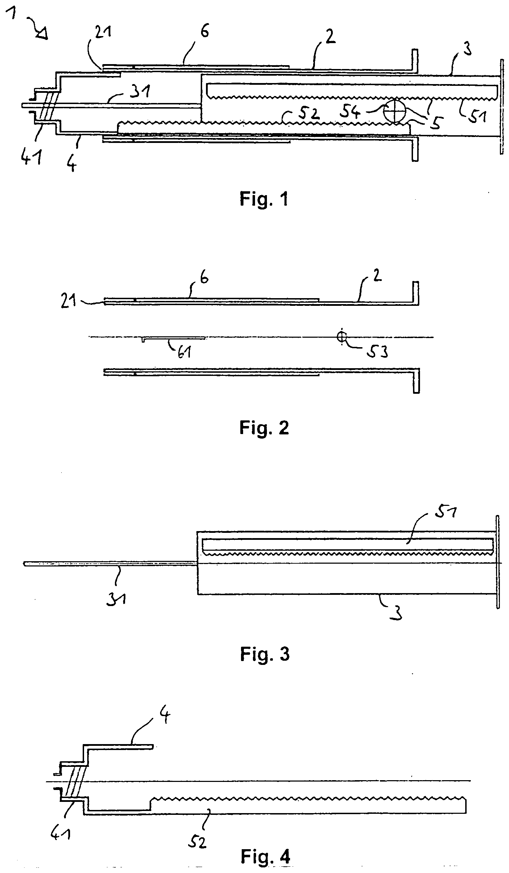

**Fig. 1** zeigt eine schematische Schnittdarstellung eines erfindungsgemäßen Applikators 1 zur Implantation eines Implantats, das hierzu aus einer Kanüle gedrückt werden kann. Eine Kanüle mit darin befindlichem Implantat (nicht dargestellt) wird mittels der in Form eines Luer-Locks ausgebildeten Kupplung 41 mit dem Applikator verbunden.

Der Applikator 1 weist einen an den Stirnseiten offenen Zylinder 2 mit einem darin verschieblich gelagertem Stempel 3 auf. An dem Stempel 3 ist ein Druckstempel 31 angeformt, der zum Hinausdrücken des Implantats aus einer Kanüle dient.

Die Kanüle wird, wie schon beschrieben, an einem Zylinderabschnitt 4 über eine im Beispiel als Luer-Lock ausgebildete Kupplung 41 befestigt. Der Zylinderabschnitt 4 ist wiederum im Zylinder 2 beweglich geführt.

Ein Getriebe 5 koppelt hierbei den Stempel 3 mit dem Zylinderabschnitt 4, um eine gegenläufige Bewegung des Zylinderabschnitts 4 und damit ein Zurückziehen der an der Kupplung 41 befestigten Kanüle beim Hineindrücken des Stempels 3 in den Zylinder 2 zu erzeugen.

Das Getriebe 5 ist so ausgestaltet, dass es eine Relativbewegung des Stempels 3 gegenüber dem Zylinder 2 in eine entgegengesetzte Bewegung des Zylinderabschnitts 4 umsetzt.

Hierzu sind zwei mit einem Zahnrad 54 gekoppelte Zahnstangen 51 und 52 vorgesehen.

Eine an dem Stempel 3 angeformte erste Zahnstange 51 steht in Eingriff mit dem am Zylinder 2 drehbar an einer Achse 53 gelagerten Zahnrad 54; siehe hierzu auch **Fig. 2** und **Fig. 3** Das Zahnrad 54 steht ebenfalls in Einriff mit einer zweiten Zahnstange 52, die am Zylinderabschnitt 4 angeformt ist; siehe auch Fig. 4.

Eine Führung und um die Achse rotatorische Stabilisierung des Stempels 3 und des Zylinderabschnitts 4 erfolgt dabei durch die Formgebung der Zahnstangen 51 und 52 (plattenförmig) zusammen mit dem an der Achse 53 drehbar befestigten Zahnrad 54.

Zusätzlich kann noch ein Endanschlag vorne und hinten am Zylinder 2 vorgesehen sein, der ein ungewolltes Herausziehen des Stempels 3 aus dem Zylinder 2 verhindert.

Des weiteren ist zum Sichern der Kanüle eine den Zylinder 2 wenigstens teilweise umschließende Hülse 6 vorgesehen, wobei diese Hülse 6 um den Zylinder 2 verschieblich gelagert ist. Die Hülse 6 ist dabei soweit über das kupplungs-seitige Ende 21 des Zylinders 2 verschieblich, dass sie eine an der Kupplung 41 des Applikators 1 angekuppelte Kanüle wenigstens teilweise umschließen kann (siehe **Fig. 1** und **Fig. 2**).

Hierzu ist die Hülse 6 in einer Zwangsführung an dem Zylinder 2 geführt, wobei die Zwangsführung durch eine Ausnehmung in der Hülse 6 und eine darin in Eingriff stehende Ausformung 61 an der Außenseite des Zylinders 2 ausgebildet ist (**Fig. 2**). Nachdem der Arzt das Implantat eingepflanzt hat, kann er die beweglich gelagerte Hülse 6 zum Schutz vor Verletzungen über die Kanüle schieben und per Drehbewegung verriegeln, die Zwangsführung kann hierzu mit einem rechtwinkligen Abschnitt vorgesehen sein.

Hierdurch ist eine Schutzvorrichtung gegen Verletzungen oder Verunreinigungen beim Umgang mit der montierten Kanüle ermöglicht.

Alle Teile des erfindungsgemäßen Applikators können aus einem sterilisierten Kunststoff hergestellt sein.

### Bezugszeichenliste

- 1: Applikator

- 2: Zylinder
- 21: Kupplungs-seitiges Ende

- 3: Stempel
- 31: Druckstempel

- 4: Zylinderabschnitt
- 41: Kupplung

- 5: Getriebe
- 51: erste Zahnstange
- 52: zweiten Zahnstange
- 53: Achse
- 54: Zahnrad

- 6: Hülse
- 61: Ausformung

## Patentansprüche

1. Applikator (1) zur Implantation eines Implantats, mit einem Zylinder (2), einem darin verschieblich gelagerten Stempel (3) und einem Zylinderabschnitt (4), welcher Stempel (3) einen Druckstempel (31) zum Hinausdrücken des Implantats aus einer Kanüle aufweist, wobei die Kanüle an einer Kupplung (41) des Zylinderabschnitts (4) befestigbar ist, wobei der Zylinderabschnitt (4) im Zylinder (2) beweglich geführt ist, wobei ein Getriebe (5) vorgesehen ist, das den Stempel (3) mit dem Zylinderabschnitt (4) derart koppelt, dass eine Relativbewegung des Stempels (3) gegenüber dem Zylinder (2) in eine entgegengesetzte Bewegung des Zylinderabschnitts (4) umgesetzt wird,
**dadurch gekennzeichnet,**
**dass** eine den Zylinder (2) wenigstens teilweise umschließende Hülse (6) vorgesehen ist, wobei die Hülse (6) um den Zylinder (2) verschieblich gelagert ist und soweit über das kupplungsseitige Ende (21) des Zylinders (2) verschieblich ist, dass sie eine an der Kupplung (41) des Applikators (1) angekuppelte Kanüle wenigstens teilweise umschließt.

2. Applikator nach Anspruch 1,
**dadurch gekennzeichnet,**
**dass** die Hülse (6) in einer Zwangsführung an dem Zylinder (2) geführt ist, wobei die Zwangsführung insbesondere durch eine Ausnehmung in der Hülse und eine darin in Eingriff stehende Ausformung (61) an der Außenseite des Zylinders (2) ausgebildet ist.

3. Applikator nach Anspruch 1 oder 2,
**dadurch gekennzeichnet,**
**dass** das Getriebe (5) eine mit dem Stempel (3) verbundene oder angeformte erste Zahnstange (51) umfaßt, die in Eingriff mit einem am Zylinder (2) drehbar an einer Achse (53) gelagerten Zahnrad (54) steht, welches ebenfalls in Eingriff mit einer zweiten Zahnstange (52) steht, die am Zylinderabschnitt (4) befestigt oder angeformt ist.

4. Applikator nach einem der Ansprüche 1 bis 3,
**dadurch gekennzeichnet,**
**dass** die Kupplung (41) durch einen Luer-Lock ausgebildet ist.

5. Applikator nach einem der Ansprüche 1 bis 4,
**dadurch gekennzeichnet,**
**dass** alle Teile des Applikators aus einem sterilisierten Kunststoff hergestellt sind.

## Claims

1. An applicator (1) for implantation of an implant, having a cylinder (2), a plunger (3) displaceably mounted therein, and a cylinder part (4), which plunger (3) has a pusher plunger (31) for pushing the implant out of a cannula, wherein the cannula is mountable on coupling (41) of a cylinder part (4), the cylinder part (4) being movably guided in the cylinder (2), there being provided a gear mechanism (5) which couples the plunger (3) to the cylinder part (4), a movement of the plunger (3) relative to the cylinder (2) being converted into an opposite movement of the cylinder part (4), **characterized in that**
a sleeve (6) that at least partially surrounds the cylinder (2) is provided, the sleeve (6) being displaceably mounted around the cylinder (2) and being displaceable beyond the coupling-side end (21) of the cylinder (2) until it at least partially surrounds a cannula coupled to the coupling (41) of the applicator (1).

2. The applicator according to claim 1, **characterized in that**
the sleeve (6) is guided on the cylinder (2) in a constraining guide means, the constraining guide means being formed especially by a recess in the sleeve and a projection (61) on the outside of the cylinder (2) that engages therein.

3. The applicator according to claim 1 or 2, **characterized in that**
the gear mechanism (5) comprises a first toothed rack (51) joined to or formed on the plunger (3), which toothed rack is in engagement with a toothed wheel (54) rotatably mounted on a shaft (53) on the cylinder (2), which toothed wheel is also in engagement with a second toothed rack (52) which is mounted or formed on the cylinder part (4).

4. The applicator according to any one of claims 1 to 3, **characterized in that**
the coupling (41) is formed by a Luer lock.

5. The applicator according to any one of claims 1 to 4, **characterized in that**
all parts of the applicator are produced from a sterilised plastics material.

## Revendications

1. Applicateur (1) pour l'implantation d'un implant, doté d'un cylindre (2), d'un poinçon (3) déposé de manière coulissante à l'intérieur dudit cylindre, et d'un tronçon de cylindre (4), lequel poinçon (3) présente un poussoir (31) pour pousser l'implant à l'extérieur d'une canule, dans lequel la canule peut être fixée à un raccord (41) du tronçon de cylindre (4), dans lequel le tronçon de cylindre (4) est guidé de manière mobile dans le cylindre (2), dans lequel est prévue une transmission (5) qui raccorde le poinçon (3) au tronçon de cylindre (4) de telle sorte qu'un mouvement relatif du poinçon (3) par rapport au cylindre (2) est converti en un mouvement opposé du tronçon de cylindre (4),
**caractérisé en**
**ce que** est prévue une gaine (6) entourant au moins partiellement le cylindre (2), dans lequel la gaine (6) est déposée de manière coulissante autour du cylindre (2) et peut être coulissée par-dessus l'extrémité du côté du raccord (21) du cylindre (2) jusqu'à ce qu'elle entoure au moins partiellement une canule raccordée au raccord (41) de l'applicateur (1).

2. Applicateur selon la revendication 1,
**caractérisé en**
**ce que** la gaine (6) est guidée sur le cylindre (2) par un guidage forcé, dans lequel le guidage forcé est formé sur le côté extérieur du cylindre (2) en particulier par un évidement dans la gaine et une déformation (61) venant en prise à l'intérieur de ladite gaine.

3. Applicateur selon la revendication 1 ou 2,
**caractérisé en**
**ce que** la transmission (5) comprend une première crémaillère (51) reliée ou moulée avec le poinçon (3), laquelle première crémaillère (51) vient en prise avec une roue dentée (54) déposée contre le cylindre (2) de manière pivotante autour d'un axe (53), laquelle roue dentée vient également en prise avec une seconde crémaillère (52) qui est fixée ou moulée contre le tronçon de cylindre (4).

4. Applicateur selon l'une des revendications 1 à 3,
**caractérisé en**
**ce que** le raccord (41) est formé par un Luer Lock.

5. Applicateur selon l'une des revendications 1 à 4,
**caractérisé en**
**ce que** toutes les pièces de l'applicateur sont fabriquées dans une matière synthétique stérilisée.
